# EUROPÄISCHE PATENTSCHRIFT

(11) **EP 2 531 823 B1**
(45) Veröffentlichungstag und Bekanntmachung des Hinweises auf die Patenterteilung: **13.11.2019**
(21) Anmeldenummer: 11702982.7
(22) Anmeldetag: 04.02.2011
(51) Int. Cl.: G01F 23/26

(54) **SENSORSYSTEM ZUR FÜLLSTANDSDETEKTION EINES FLUIDS IN EINEM GEFÄSS**
SENSOR SYSTEM FOR DETECTING THE FILL LEVEL OF A FLUID IN A VESSEL
SYSTÈME CAPTEUR POUR LA DÉTECTION DU NIVEAU D'UN FLUIDE DANS UN RÉCIPIENT

(30) Priorität: 04.02.2010 DE 102010001605
(43) Veröffentlichungstag der Anmeldung: 12.12.2012
(60) Teilanmeldung: 19188600.1
(73) Patentinhaber: Fresenius Medical Care Deutschland GmbH, 61352 Bad Homburg (DE)
(72) Erfinder: SCHNEIDER, Jochen, 97537 Wipfeld (DE); GAGEL, Alfred, Litzendorf 96123 (DE)
(74) Vertreter: Nordmeyer, Philipp Werner
(86) Internationale Anmeldenummer: PCT/EP2011/051623
(87) Internationale Veröffentlichungsnummer: WO 2011/095573

(56) Entgegenhaltungen:
- WO-A1-00/42395
- WO-A1-2006/021295
- DE-A1- 19 949 985
- DE-A1-102005 057 558
- GB-A- 1 472 025
- US-A- 4 749 988

## Beschreibung

Die Erfindung bezieht sich auf ein Sensorsystem zur kapazitiven Füllstandsmessung eines flüssigen Mediums nach dem Oberbegriff des Anspruchs 1 und ein medizinisches Gerät nach Anspruch 16.

In diversen Anwendungen werden Sensoren benötigt, um den Füllstand von Flüssigkeiten, insbesondere elektrisch leitenden Flüssigkeiten, zu messen. Wenn hohe Anforderungen an die Reinheit der Flüssigkeit und Schutz vor Verschmutzungen gestellt werden und die Flüssigkeit nicht in Kontakt mit der Messapparatur kommen soll, eignen sich nicht-invasive Messsysteme, die etwa mit einem elektrischen Feld und dessen Beeinflussung durch das zu messende Medium die Füllstandserkennung durchführen.

Aus DE19949985A1 ist ein kapazitiver Füllstandssensor bekannt, bei dem eine erste Messelektrode an einer Seitenwand eines Behälters befestigt ist, welche über einen Verstärker mit einer Spannung einer bestimmten Frequenz beaufschlagt wird, so dass sich die elektrischen Feldlinien gemäß einem Kondensator zu der zweiten Elektrode aufbaut die unterhalb des Behälters angeordnet ist. Bei z.B. einer Vergrößerung der Behälterkapazität C (z.B. durch die Temperaturabhängigkeit der Dielektrizitätszahl ε_{R}) kann das Messergebnis verfälscht werden. Aus diesem Grund ist eine Kompensationselektrode C an der Behälterwand so angeordnet, dass die Feldlinien im Wesentlichen von dieser Elektrode ausschließlich durch die Wandstärke des Behälters laufen und so deren Einfluss erkennen.

Neben der Beeinflussung des detektierten Füllstands z.B. durch sich verändernde Eigenschaften der Behälterwand, stellt vor allem die Leitfähigkeit des zu messenden Mediums in Kombination der Benetzungsfähigkeit der Behälteroberfläche und der damit verbundenen Filmbildung an der Innenseite des Behälters beim Absinken des Pegels eine Herausforderung für die Messapparatur dar. Auch müssen mögliche schwallartige Pegeländerungen berücksichtigt werden. Ebenso stellt die Koppelung ein Problem dar. Die Koppelung ist der Grad für die Qualität der Aufnahme des Behälters in der Messapparatur. An der Übergangstelle von dem Behälter zu der Aufnahme ergeben sich Koppelkapazitäten C_{K}. Die Impedanz Z_{C} dieses Übergangs wird wesentlich durch den kapazitiven Blindwiderstand X_{C} = 1/ωC = 1 / 2π f C_{K} ausgedrückt.

Die Messstrecke zwischen den Kondensatorplatten der Messapparatur wird als eine Reihenschaltung von Plattenkondensatoren gesehen, wobei sich die Kapazität eines Plattenkondensators berechnet als: C = ε₀ * εᵣ *A / d
ε₀ ist die Dielektrizitätskonstante (elektr. Feldkonstante), A die wirksame Fläche und d der entsprechende Plattenabstand, welcher dem Verlauf der Feldlinien entspricht. εᵣ ist die Dielektrizitätszahl, bzw. relative Permittivität des Mediums. Bei einem relevanten Messmedium, z.B. Blut, isotonische Kochsalzlösungen o.ä. besteht eine sehr starke Frequenzabhängigkeit der Dielektrizitätszahl εᵣ. Dagegen ist die spezifische Leitfähigkeit κ der relevanten Medien mit κ= 6 (Blut) oder κ=16 (NaCl) mS/cm nur in einem geringen Maße frequenzabhängig.

In der später noch im Detail beschriebenen Messapparatur bewegen sich die Koppelkapazitäten im pF-Bereich. Der ohmsche Widerstand des Mediums liegt dabei im einstelligen kΩ-Bereich. Aus dieser Abschätzung wird klar, dass bei kapazitiven Sensoren das Messergebnis mit einer Arbeitsfrequenz f im kHz-Bereich bis zum einstelligen MHz-Bereich im Wesentlichen durch die Koppelkapazitäten bestimmt wird, da die Impedanz und die Phasenlage der Anordnung durch deren Blindwiderstand dominiert wird.

Bekannte Messapparaturen sind nicht in der Lage, zuverlässig zwischen einem realen Füllstand und nur einem dünnen Flüssigkeitsfilm bzw. -schwall zu unterscheiden, da der nur geringe Einfluss des Unterschieds im Ohmschen Widerstand eines massiven Mediums zu dem eines dünnen Flüssigkeitsfilms bereits durch kleinste Veränderungen in der Koppelung überdeckt wird.

So ergibt sich die Notwendigkeit einer hohen Arbeitsfrequenz, wie z.B. größer 75 MHz, wie es unter DE19651355 A1 oder D E 10 2005 057 558 erläutert wird. Andererseits stellen hohe Arbeitsfrequenzen, also z.B. 80MHz oder im dreistelligen MHz-Bereich hohe Anforderungen an das Geräte- und Schaltungsdesign und insbesondere dessen EMV-konforme Gestaltung.

Aufgabe der vorliegenden Erfindung ist, eine nichtinvasive Füllstandserkennung im Sinne "Level" - "NoLevel", also der binären Information, ob ausreichender Füllstand von wässrigen Medien wie z.B. Blut, Kochsalzlösung, Dialysat o.ä. in einem nicht leitfähigen Behälter vorhanden oder nicht. Die Zielsetzung ist dabei:
- Das Sensorsystem soll eine Filmbildung an der Innenwand des Behälters erkennen können bzw. gegen den wirklichen Füllstand auch im Fall eines Pegelabfalls unterscheiden können.
- Das Sensorsystem soll robust bezüglich einer möglichen Schwallbildung im Bereich der Sensorelemente sein.
- Das Sensorsystem soll möglichst zwischen Blut und einem dichten Schaum, wie einem Blut-Luft-Gemisch unterscheiden können. Ein derartiges Blut-Luft-Gemisch kann sich einem venösen Blasenfänger bei Lufteintritt in den extrakorporalen Kreislauf ausbilden und sammelt sich insbesondere an der Oberfläche des zu messenden Mediums an.
- Das Sensorsystem soll möglichst robust gegenüber Berührungen sein und die Pegelerkennung sollte zuverlässig für ein geerdetes sowie ein nicht mit (Funktions-)erde verbundenem Medium funktionieren.
- Das Sensorsystem soll in der Lage sein, eine möglicherweise nicht korrekte Aufnahme des Behälters in der Aufnahme des Sensorsystems zu erkennen und insbesondere fortlaufend die korrekte Aufnahme überwachen können.
- Das Sensorsystem soll robust gegenüber Verunreinigungen wie z.B. Feuchtigkeitsansammlungen im Messraum, wie im Falle einer Leckage, sein und diese erkennen können. Bei dem später näher beschriebenen erfinderischen Behälter umfasst der Messraum den Bereich außerhalb des Behälters und einer Aufnahme, welche die Sensoren aufweist.

Diese Aufgabe wird durch die Merkmale des Anspruchs 1 gelöst. Die nachfolgenden abhängigen Ansprüche beschreiben vorteilhafte Weiterbildungen.

Erfindungsgemäß wird ein Sensorsystem zur kapazitiven Messung des Füllstands eines flüssigen Mediums in einem Behälter, vorzugsweise einem Blasenfänger eines Dialysegeräts, bereitgestellt, welches eine Aufnahme aufweist, an welcher zwei Kontaktbereiche zum Kontakt mit einer Außenfläche des aufgenommenen Behälters vorgesehen sind. Dabei befindet sich zumindest je eine Pegelerkennungselektrode in den Kontaktbereichen zur kapazitiven Erkennung des Flüssigkeitspegels im Behälter. Dabei weist das Sensorsystem eine Koppelungsmessvorrichtung zur kapazitiven Bestimmung des ordnungsgemäßen Ankoppelns des Behälters in der Aufnahme auf. Das Koppeln entspricht insbesondere einem mechanischen Kontakt, wobei auch bei einem verbleibenden (kleinen) Luftspalt von einer Koppelung ausgegangen wird.

Ein Vorteil dieses Aufbaus ist, dass er nicht-invasiv ist. Das heißt, die Messung wird durchgeführt, ohne dass Messsensoren oder -fühler in Kontakt mit der Flüssigkeit kommen. Das Sensorsystem ist also eine Art Adapter, welcher an dem Behälter befestigt werden kann. Mangelnde Koppelung, wie z.B. mangelnder mechanischer Kontakt kann durch die kapazitive Koppelungsmessung erkannt werden. Da eine gemeinsame Auswerteelektronik für die genannten kapazitiven Messungen verwendet werden kann, werden Kosten gespart. Die getrennte Koppelungsmessung erlaubt, die Füllstandsmessung mit höheren Frequenzen zu betreiben, ohne die Gefahr zu bewirken, dass aufgrund der hohen Frequenzen die Füllstandsmesswerte bei unzureichender Kopplung verfälscht werden.

Vorteilhafterweise weisen Pegelerkennungselektroden geteilte Elektrodenbereiche auf und die Koppelungsmessvorrichtung nimmt die Koppelung über eine kapazitive Messung über die Elektrodenbereiche der jeweiligen Pegelerkennungselektrode vor. Durch die unterschiedliche Beschaltung wird in jedem Kontaktbereich nur eine geteilte Elektrode verwendet, wodurch Komponentenkosten gespart werden. Auch kann das Sensorsystem konstruktiv derart ausgelegt werden, dass dieses Elektrodenpaar möglichst exakt und unmittelbar im genannten Kontaktbereich angeordnet ist, dass an der gleichen Stelle beide Messungen durchgeführt werden können.

In einer alternativen Ausführung weist die Kopplungsmessvorrichtung an jedem Kontaktbereich zwei Koppelelektroden auf, die sich von den Pegelerkennungselektroden unterscheiden. Insbesondere sind dabei die Koppelelektroden axial in Längsrichtung des Behälters gegen die Pegelerkennungselektroden versetzt. Hierdurch wird eine funktionale Trennung erreicht und ermöglicht, dass zeitgleich die genannten unterschiedlichen Messungen durchgeführt werden können. Auch wird kein Fokus-Array (wie später beschrieben wird) zur Umschaltung der Anbindung der Elektroden an den Analysator benötigt.

Weiterführend ist die Koppelungsmessvorrichtung geeignet, sowohl eine zu geringe Ankoppelung, wie etwa im Fall eines Abstands von dem Behälter zu der Aufnahme, als auch eine zu hohe Ankoppelung, wie etwa im Fall von Flüssigkeiten oder sonstigen Fremdsubstanzen in den Kontaktbereichen, zu erkennen. Durch die doppelte Funktion der Koppelungsmessung wird so einfach und kostengünstig auch erkannt, ob die Koppelung durch Verunreinigungen zu groß wird, so dass ein fälschlicherweise positives Ergebnis der Pegelerkennungsmessung vermieden bzw. erkannt werden kann.

In einer Ausgestaltung ist die Koppelungsmessvorrichtung geeignet, im Fall, dass die Messung des Flüssigkeitspegels im Behälter einen ausreichenden Pegel erkennt und gleichzeitig die Kopplungsmessung eine unzureichende Kopplung erkennt, einen Voralarm auszulösen, um den Anwender auf eine zu geringe Kopplung hinzuweisen. Da bei einem positiven Ergebnis der Pegelerkennungsmessung bei unzureichender Koppelung, das Ergebnis der Pegelerkennung unkoordiniert und ohne erkennbare äußere Einflüsse bei unverändertem wirklichen Pegel umspringen kann, kann dieses Problem durch eine rechtzeitige Warnung der mangelnden Kopplung vermieden werden. Der Voralarm bzw. das Anzeigen einer mangelnden Kopplung beugt auch einer Fehlinterpretation der sonst unspezifischen "NoLevel"-Meldung vor, welche in der Praxis häufig zu einer Überfüllung des Blasenfängers führt.

In einer weiteren Ausgestaltung kann zur Blasenfängererkennung über die Koppelungsmessung kontrolliert werden, ob der Bediener den leeren Blasenfänger richtig eingelegt hat.

Insbesondere wird über die Pegelerkennungselektroden eine komplexe Gesamtimpedanz Z_{ges} bestimmt und über diese die Pegelerkennung durchgeführt. Vorzugsweise wird dabei ihr Realteil ausgewertet. Da der Realteil ein Maß für den elektrischen Widerstand in der Messflüssigkeit ist und der Widerstand bei einem Film an der Innenwand des Gefäßes oder einem Schwall höher ist, als bei einem entsprechenden Füllstand, kann auf diese Weise einfach und sicher die Messung durchgeführt und zwischen einem Flüssigkeitsfilm oder entsprechendem Füllstand unterschieden werden. Bei Sensoren nach dem Stand der Technik wird dagegen im Wesentlichen die Phasenlage der komplexen Gesamtimpedanz Z_{ges} ausgewertet, wobei die Pegelerkennung, also die Level / NoLevel-Information über das Anschwingen bzw. das Nicht-Anschwingen des Oszillators abgeleitet wird. Dies kann auch als eine grobe Quantisierung der Phaseninformation aufgefasst werden. Durch die Auswertung des Realteils wird der Füllstand genauer erkannt und der Realteil kann ebenfalls optional (wie später beschrieben wird) für die Erkennung von dichtem Schaum verwendet werden. Entsprechend kann durch die Auswertung des Realteils eine weniger hohe Arbeitsfrequenz verwendet werden.

In einer Weiterführung der Erfindung wird über die Pegelerkennungselektroden eine komplexe Gesamtimpedanz Z_{ges} bestimmt und über einen Demodulator zeitliche Variationen der Gesamtimpedanz Z_{ges} bestimmt und in einer Auswerteeinheit zusammen mit der Gesamtimpedanz Z_{ges} zur Bestimmung der Pegelerkennung eingesetzt, um so Inhomogenitäten im zu messenden Medium wie z.B. einen Blasenanteil zu erkennen. Anders ausgedrückt geht es hierbei um die Erfassung der komplexen Impedanz Z_{ges} (Betrag und Phase bzw. Imaginär- und Realteil) mit hoher Auflösung über die Zeit. Da Blasen ihre Lage an der Oberfläche des Flüssigkeitsspiegels der Messflüssigkeit häufig ändern - insbesondere wenn der Behälter ein Blasenfänger ist - verändern sich durch den untergemischten ungelösten Luftanteil die elektrischen Eigenschaften des vom Messfeld durchdrungenen Mediums mit einer deutlich höheren Frequenz, als dies durch ein Befüllen oder Entleeren passieren könnte. Da erkannt wurde, dass derartig schwankende Messergebnisse durch Blasen entstehen, können diese Messergebnisse für eine entsprechende Auswertung verwendet werden.

Beispielsweise wird die Messung der Pegelerkennung mit einer Frequenz größer als 60 MHz, vorzugsweise größer als 70 MHz durchgeführt. Der wesentliche Zweck einer hohen Arbeitsfrequenz ist es dabei, den Anteil der Impedanz der Koppelkapazität an der Gesamtimpedanz soweit zu reduzieren, dass sich die näherungsweise frequenzunabhängigen Unterschiede im ohmschen Widerstand des zu messenden Mediums und eines nur dünnen Films deutlich abheben und erfasst werden können.

Weiter wird in einer Ausführungsform die Messung der Pegelerkennung mit einer Frequenz kleiner als 90 MHz, insbesondere kleiner als 78 MHz durchgeführt. Bei Messsystemen, bei denen die Arbeitsfrequenz sehr groß ist, bspw. im Bereich von 100 MHz oder mehr, kann dadurch zwar zuverlässig zwischen einem Film vom Medium an der Innenoberfläche des Behälters und dem Füllstand des zu messenden Mediums unterschieden werden. Diese System büßen dafür allerdings die Möglichkeit ein, noch vor einem Befüllen die korrekte Koppelung zwischen Sensorik und zu überwachenden Behälter überprüfen zu können. Eine hohe Arbeitsfrequenz ist in diesem Sinne für die Bestimmung der Kopplung eher hinderlich. Hierbei wird insbesondere für die Messung Pegelerkennung und der Kopplungsüberwachung jeweils eine Frequenz eines optimalen Arbeitspunkts definiert und die Messung wird durch eine Umschaltung der Frequenzen vorgenommen.

Es sind Mittel zur Erzeugung von elektrischen Feldern für die Pegel- und Koppelungsmessung und Mittel zur Erfassung der komplexen Impedanz unterschiedlicher Messelektroden (C11, C12, C21, C22) nach Betrag, Phase und deren zeitlicher Änderung vorhanden. Dabei kann das System abhängig von einem Ansteuersignal einen Netzwerkanalysator mit unterschiedlichen Messelektroden des Sensorsystems verbinden. Dieses Messsystem ist dabei insbesondere eingerichtet, eine komplexe Impedanz mit hoher Auflösung zu messen. Ein Fokus-Array wird dabei insbesondere als ein elektronischer Logikschaltkreis verstanden, welcher abhängig von seiner Ansteuerung bestimmte Eingangssignale an bestimmte Ausgangsports weiterleitet. Auf diese Weise kann mit einfachen Mitteln eine einzige Auswerteelektronik wahlweise an unterschiedliche Elektroden für die genannten Messungen angeschlossen werden.

Der Behälter kann ein Blasenfänger eines Dialysegeräts sein. Gerade in diesem Anwendungsfall wird eine Messung von Flüssigkeitsständen in einem Behälter benötigt, da stets ein bestimmter Pegel für die zuverlässige Funktion des Geräts notwendig ist. Dieser Pegel kann ein Mindestpegel, wie z.B. eine Teilfüllung, oder bei einem luftfreien Kreislauf ein möglichst vollständig gefüllter Blasenfänger sein. Der Behälter kann ebenfalls ein Blasenfänger einer medizinischen Infusions- oder Transfusionsapparatur sein.

In einem entsprechenden Verfahren zum kapazitiven Messen des Füllstands eines flüssigen Mediums in einem Behälter wird der Behälter in einer Aufnahme des Sensorsystems aufgenommen und eine Pegelerkennungsvorrichtung des Sensorsystems ermittelt kapazitiv den Flüssigkeitspegel im Behälter, und mit einer kapazitiven Koppelungsmessvorrichtung wird das ordnungsgemäße Ankoppeln des Behälters in der Aufnahme bestimmt. Hierbei kann die Koppelungsmessvorrichtung auch auf die Messergebnisse der Pegelerkennung zurückgreifen, um so sicher die ordnungsgemäße Koppelung zu erkennen und entsprechend kann bei der Pegelerkennung zur Erhöhung der Messgenauigkeit auf die Messergebnisse der Koppelungsmessung zurückgegriffen werden.

Vorteilhafter Weise werden die Pegelerkennungsmessung und die Koppelungsmessung zeitlich versetzt durchgeführt. Hierdurch können Störungen der bei den Messungen verwendeten elektrischen Felder vermieden werden und ferner können insbesondere für die unterschiedlichen Messungen die gleichen Elektroden verwendet werden, welche mit unterschiedlichen Frequenzen betrieben werden. Ferner können über die zeitlichen Veränderungen der Messergebnisse Rückschlüsse auf eine mögliche Blasenbildung im Messmedium gezogen werden.
Ein medizinisches Gerät, insbesondere ein Dialysegerät, weist ein entsprechendes Sensorsystem und einen Flüssigkeitskreislauf eines elektrisch leitenden wässrigen Mediums mit einem Blasenfänger auf. Dabei ist das Sensorsystem eingerichtet, den Flüssigkeitsstand in dem Blasenfänger zu messen und abhängig vom Messergebnis eine Förderpumpe oder ein Ventil oder Klappe des Flüssigkeitskreislaufs zu steuern.

Das Dialysegerät ist bevorzugt ein Hämodialysegerät zur Entfernung harnpflichtiger Substanzen aus dem Blut eines an Niereninsuffizienz leidenden Patienten mit einem extrakorporalen Blutkreislauf, der über einen Blasenfänger verfügt, oder das erfindungsgemäße Sensorsystem angekoppelt wird.

Anhand der Zeichnung wird die Erfindung nachstehend eingehend erläutert. Es zeigen:
- Fig. 1: einen Querschnitt durch die Messapparatur mit den Feldlinien der Messung der Pegelerkennung,
- Fig. 2: ein Ersatzschaltbild der Messapparatur,
- Fig. 3: ein Schema der Messvorrichtung,
- Fig. 4: einen weiteren Querschnitt durch den Behälter mit den alternativen Elektroden und Feldlinien der Pegelerkennung,
- Fig. 5: einen Querschnitt durch die Messapparatur mit Feldlinien der Koppelungsmessung,
- Fig. 6: einen Querschnitt durch die Messapparatur mit Fremdstoffen in den Kontaktbereichen von der Aufnahme zu dem Behälter,
- Fig. 7: ein zeitliches Diagramm des Ergebnisses der Modulatorauswertung,
- Fig. 8: einen Querschnitt durch den Behälter mit Andruckhebeln und
- Fig. 9: alternative Ausgestaltungen der Elektroden.

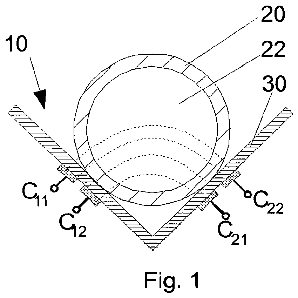

Fig. 1 zeigt einen Querschnitt durch einen zylindrischen Behälter 20, der innen einen Flüssigkeitsbereich 22 aufweist. Im üblichen Einbau ist der Behälter 20 vertikal ausgerichtet, so dass sich die Flüssigkeit in seinem unteren Bereich sammelt. Der dargestellte Schnitt liegt entsprechend in der horizontalen Ebene. An zwei Umfangsseiten besteht Linienkontakt von dem Behälter mit einer Aufnahme 30 des Sensorsystems 10. "Linienkontakt" bedeutet, dass durch die Deformation des weichen Materials des Blasenfängers auch ein schmaler flächiger Kontakt vorhanden sein kann. Nicht dargestellte Andruckmittel 24 sorgen dafür, dass ein sicherer mechanischer Kontakt von dem Behälter zu der Aufnahme gewährleistet ist. Im Bereich der beschriebenen Kontaktfläche sind Kondensatorplatten C₁₁, C₁₂, C₂₁ und C₂₂ angeordnet. Dabei sind in der Messung der Pegelerkennung über die Ansteuerungselektronik die Platten C₁₁ und C₁₂ unmittelbar und direkt miteinander verbunden. Entsprechend sind die Elektroden C₂₁ und C₂₂ verbunden. Bei Beaufschlagung der Kondensatorplatten mit einer elektrischen Spannung bzw. Frequenz ergeben sich die dargestellten elektrischen Feldlinien. Hierbei sind die Platten derart angeordnet, dass möglichst viele Feldlinien durch den Flüssigkeitsbereich geleitet werden. Dabei besteht zwischen den beiden Flanken der Kontaktflächen der Aufnahme 30 ein Winkel von 90°. Winkelbereiche von 60° bis 150° sind vorteilhaft. Die Kontaktflächen können in beliebigen Winkeln zueinander stehen. Bei beispielsweise 180° zwischen den Kontaktflächen liegen diese in einer Ebene, und der Blasenfänger wird mechanisch an die Kontaktflächen gepresst. Bei beispielsweise 360° zwischen den Kontaktflächen liegen sich diese gegenüber, und der Blasenfänger befindet sich in diesem Falle zwischen den Kontaktflächen. Durch die Messung des Feldes kann, wie später im Detail beschrieben, der Füllstand bestimmt werden.

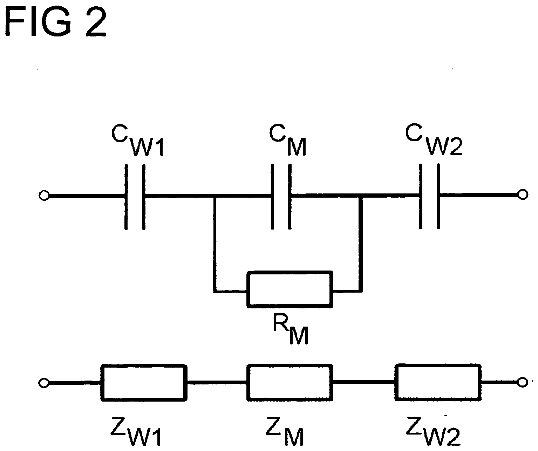

Fig. 2 zeigt das Ersatzschaltbild für die Pegelerkennungsmessung. Hierbei sind drei Kondensatoren, also die Kapazität C_{W1} der ersten Wand des Behälters, welche durch Feldlinien durchdrungen wird, dann die Kapazität C_{M} des zu messenden Mediums und die Kapazität C_{W2} der zweiten Wand in Reihe geschaltet. Da das zu messende Medium elektrisch leitend ist, ist der Widerstand R_{M} des Mediums parallel zu C_{M} geschaltet. Die Gesamtimpedanz Z_{ges} ergibt sich als: Z_{ges} = Z_{W1} + Z_{M} + Z_{W2}. Der Einfachkeit halber wird bei obiger Betrachtung die Kapazität der Wand der Aufnahme 30 mit der Kapazität der entsprechenden Wand des Behälters gemeinsam betrachtet.

Eine ebenfalls alternative Ausführungsform kann vorsehen, dass sich auf der Innenseite der Aufnahme jeweils eine metallisierte oder metallene Flache gegenüber den sich auf der Außenseite der Aufnahme befindlichen Kondensatorflächen C₁₁, C₁₂, C₂₁ und C₂₂ befindet. Diese metallisierten oder metallenen Flächen stehen direkt mit dem Behälter 20 in Kontakt und bilden mit den Kondensatorplatten C₁₁, C₁₂, C₂₁ und C₂₂ und dem Messmedium im Blasenfänger jeweils eine Reihenschaltung zweier Kondensatoren. Die kapazitive Kopplung an den Blasenfänger wird dadurch verbessert. Die elektrische Kontaktierung findet bei dieser Ausführungsform auf der dem Behälter 20 gegenüberliegenden Seite an den Kondensatorplatten C₁₁, C₁₂, C₂₁ und C₂₂ statt.

In einer alternativen Ausführung können - im Gegensatz zu z.B. Fig. 1 - die Kondensatorplatten C₁₁, C₁₂, C₂₁ und C₂₂ nicht auf der dem Behälter gegenüberliegenden Seite der Aufnahme 30 angeordnet sein, sondern können auf der Innenseite der Aufnahme liegen, so dass die Kondensatorplatten direkt mit dem Behälter 20 in Kontakt stehen. Hierdurch wird ein Einfluss durch die Wandstärke und -material der Aufnahme 30 minimiert.

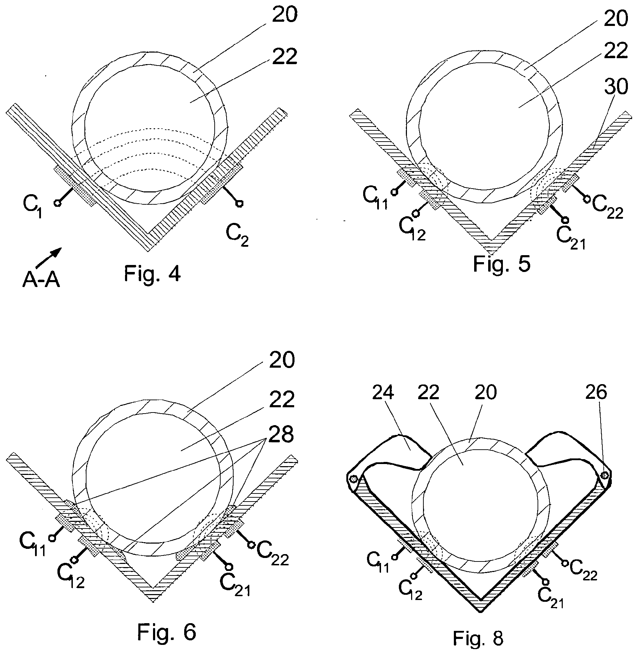

Fig. 5 zeigt die Feldlinien einer alternativen Beschaltung der Kondensatorflächen C₁₁, C₁₂, C₂₁ und C₂₂, in dem Sinne, dass das elektrische Feld über jeweils zwei benachbarte Kondensatorflächen C₁₁ und C₁₂, bzw. C₂₁ und C₂₂ aufgebaut wird. Hierdurch kann, wie später noch beschrieben wird, die korrekte Ankoppelung, also Berührung des Behälters 20 in der Aufnahme 30 erkannt werden. Bei der bislang beschriebenen Ausführung werden also die gleichen Elektroden einmal für die Pegelerkennung und davon getrennt in einem zeitlich versetzen Messschritt zur Überprüfung der Kopplung eingesetzt. Die Fläche der Kondensatorflächen C₁₁, C₁₂, C₂₁ und C₂₂ beträgt jeweils 7 * 10 mm und der Spalt zwischen den benachbarten Kondensatorflächen beträgt 1 bis 2 mm. Die Kondensatorflächen und der Spalt können auch andere Formen und Abmaße aufweisen. Das bedeutet, dass die Kondensatorfläche der gemeinsamen Beschaltung gemäß Fig. 1 zur Pegelerkennung ca. 15 (oder 16) mm * 10 mm beträgt. Anders als bei dem Stand der Technik gemäß DE 199 49 985 A1 wird nicht in Bezug auf den Boden des Behälters gemessen, sondern in einem Abschnitt quer zur Länge des Behälters. Um die Pegelerkennung der Flüssigkeit korrekt bestimmen zu können, wird durch geeignete Befestigungsmittel (nicht dargestellt) sichergestellt, dass der Behälter 20 axial ausgerichtet in einer definierten Lage in der Aufnahme 30 aufgenommen wird.

Der Außendurchmesser eines vorzugsweise zylindrischen Behälters, wie z.B. ein Blasenfänger einer Dialysevorrichtung, beträgt bspw. 19 - 23 mm mit einer Wandstärke von bspw. 1,5 mm. Hierbei kann der Behälter, vorzugsweise aus Kunststoff, auch eine elliptische oder ovale Grundform aufweisen. Der Behälter kann auch integraler Bestandteil einer medizinischen Blutbehandlungskassette sein, wobei der Behälter beliebige Formen annehmen kann. So sind auch Behälter mit einer Mehrzahl ebener Außenflächen, wie z.B. einem quadratischen Querschnitt der Außenkontur denkbar. Aufgrund der Breite der Messelektroden, bzw. Kondensatorflächen, also in der dargestellten Schnittebene, wird eine ausreichende Kompatibilität für unterschiedlich große Behälter 20 gewährleistet.

Die bereits beschriebene komplexe Impedanz Z_{ges} lässt sich charakterisieren durch den Betrag und die Phasenlage, bzw. durch den Imaginär- und Realteil. Der eigentliche Messeffekt der Pegelerkennung liegt vorzugsweise im Realteil der Impedanz Z_{ges}. Die vorteilhafte Trennung und Erkennung vom Füllstandsverlauf in Vergleich zu einem Schwall oder Film ergibt sich durch die Unterschiede in den Leitwerten von dem Film zum Medium, was dem Realteil der Impedanz Z_{ges} entspricht. Bei eher niedrigen Arbeitsfrequenzen wird die komplexe Impedanz Z_{ges} durch den Imaginärteil dominiert. Entsprechend lässt sich die Unterscheidung zwischen einem Schwall oder Film gegen den Füllstand vor allem bei höheren Arbeitsfrequenzen, bspw. größer 80 kHz, durchführen. Aus EMV-Gründen sind Frequenzen größer als z.B. 300 MHz nachteilig.

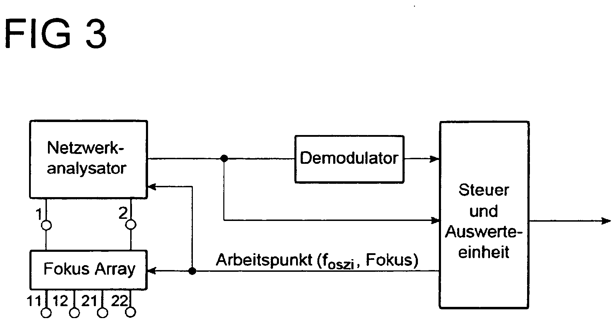

Gemäß Fig. 5 ist auf der rechten Seite, also bei den Kondensatorflächen C₂₁ und C₂₂ ein gewisser Abstand von dem Behälter 20 zu der Aufnahme 30. Dies stellt eine unzureichende Koppelung dar. So verläuft das dargestellte elektrische Feld zur Erkennung der Kopplung großteils durch die Luft und weniger durch die Wandstärke des Behälters 20. Durch eine entsprechende Auswertung des Feldverlaufs kann unzureichende Ankoppelung erkannt werden. Dabei kann, wie oben bei der Füllstandsmessung beschrieben, der Betrag der Impedanz Z₁₁ zu Z₁₂ bzw. Z₂₁ zu Z₂₂ ausgewertet werden. Alternativ kann, wie bereits aus dem Stand der Technik bekannt, die Phasenlage betrachtet werden. Die bislang beschriebenen Messungen der Koppelung bzw. der Pegelerkennung werden zeitversetzt durchgeführt. Hierfür bestimmt die in Fig. 3 dargestellte Steuer- und Auswerteeinheit unterschiedliche Arbeitspunkte und entsprechend wird die Verschaltung des Fokus-Arrays unterschiedlich gesteuert.

In einer ersten Verschaltung des Fokus-Arrays wird die Pegelerkennung durchgeführt. Dafür werden die Ports 11, 12, 21 und 22, welche mit den entsprechend bezeichneten Kondensatorflächen verbunden sind, so verschaltet, dass 11 und 12 kurzgeschlossen an den Kontakt 1 des Netzanalysators geleitet werden und die Ports 21 und 22 entsprechend kurzgeschlossen mit dem Kontakt 2 des Netzanalysators verbunden sind, so dass ein Feld gemäß Fig. 1 durch den Netzwerkanalysator erzeugt werden kann. In einer zweiten Verschaltung wird der Port 11 an den Kontakt 1 geleitet und Port 12 an Kontakt 2, so dass die Koppelung (gemäß Fig. 5 auf der linken Seite) durchgeführt wird. Die Ports 21 und 22 werden in diesem Fall nicht angeschlossen. In einer dritten Verschaltung werden die Ports 21 und 22 entsprechend mit den Kontakten 1 und 2 verbunden, so dass die Ankoppelung gemäß Fig. 5 (rechte Seite) durchgeführt werden kann. Die so von dem Netzwerkanalysator erhaltenen drei Messergebnisse werden von der Steuer - und Auswerteeinheit aufgenommen und mit einer später näher beschriebenen Auswertelogik zu den Ausgabewerten umgewandelt, welche dem Bediener die Pegellage, die Koppelung oder das Vorhandensein von Schaum signalisieren. Bei den unterschiedlichen Verschaltungen des Fokus-Arrays wird ggf. die Messung mit einer jeweils optimierten Arbeitsfrequenz durchgeführt.

Gemäß Fig. 6 ist der Fall dargestellt, dass Feuchtigkeit bzw. eine Flüssigkeit sich in den Kontaktbereichen von der Aufnahme zu dem Behälter befindet. Dadurch werden die Messergebnisse der Koppelungsmessung derart verändert, dass die Koppelung deutlich erhöht ist. Diese Feuchtigkeit ist auch schädlich, da dadurch die Füllstandsmessung negativ beeinflusst wird. Der Netzwerkanalysator erkennt diese erhöhte Koppelung und teilt dies der Steuer- und Auswerteeinheit mit, welche entsprechend, eine Warnung an den Benutzer ausgeben kann.

In der nachfolgenden Tabelle ist die logische Verschaltung der Steuer- und Auswerteeinheit dargestellt. Sie erhält das in der ersten Verschaltung erhaltene Messergebnis der Füllstandsmessung, welche mit 0 (=schlechter Level; = NoLevel) und 1 (=ausreichender Level) bezeichnet ist. Die Messergebnisse der zweiten und dritten Verschaltung werden mit einer logischen UND-Verknüpfung verbunden und als 0 (=schlechte Koppelung) und 1 (=gute Koppelung) der Auswerteeinheit zugeführt.

| Levelmessung | 0 | | | 1 | | |
|---|---|---|---|---|---|---|
| Kopplungsmessung | 0 | 1 | 2 | 0 | 1 | 2 |
| Auswerteergebnis | Alarm | Alarm | Undichtigkeit | Voralarm | Okay | Undichtigkeit |

Nur im Fall, dass die Levelmessung =1 und die Kopplungsmessung =1, wird das Auswerteergebnis als okay gesetzt, was dem Benutzer z.B. durch ein grünes Signal mitgeteilt werden kann. Wenn die Levelmessung gleich 0 ist, wird anhand des Maschinenzustands eine weitere Abstufung vorgenommen: Im Betriebszustand der Dialyse oder dem Spülen wird bei guter Kopplung (=1) ein Alarm eines abgefallenen Pegels ausgegeben und bei schlechter Kopplung (=0) eine Warnung der möglicherweise fehlerhaften Ankopplung bzw. Position des Blasenfängers ausgegeben. Im Betriebszustand des Aufrüstens wird keine Aktion durchgeführt, da ggf. kein Blasenfänger aufgenommen ist und somit auch kein Pegel erwartet wird bzw. auch nicht vorhanden sein kann. Im Betriebszustand nach dem Aufrüsten und beim Füllen wird zumindest ein korrekt eingelegter Blasenfänger erwartet und bei guter Kopplung (=1) kein Alarm, bei schlechter Kopplung (=0) aber eine entsprechende Warnung ausgegeben.

Wenn die Levelmessung gleich 1 und die Koppelung gleich 0, so ergibt sich der Fall eines Voralarms. Da die Levelmessung ein positives Signal ergibt, muss kein Alarm ausgegeben werden. Allerdings ist es möglich, dass zu einem späteren Zeitpunkt bei weiterhin gutem Level der Flüssigkeit selbst, das Ergebnis der Levelmessung aufgrund der schlechten Koppelung auf 0 (= NoLevel) springt und so einen schlechten Level signalisiert, welcher eigentlich durch die Koppelung verursacht wurde. Durch den Voralarm wird dem Benutzer eine Vorwarnung gegeben, mit der Aufforderung die Koppelung zu verbessern. So können Fehlalarme, welche durch schlechte Koppelung verursacht sind, oder vor allem auch Fehlinterpretationen des Messergebnisses durch den Anwender vermieden werden.

Ferner wurde beschrieben, dass die Koppelungsmessung eine erhöhte Koppelung erkennen kann. Diese ist in obiger Tabelle mit 2 bezeichnet und bewirkt eine Warnmeldung einer Undichtigkeitsanzeige unabhängig von dem Wert der Levelmessung.

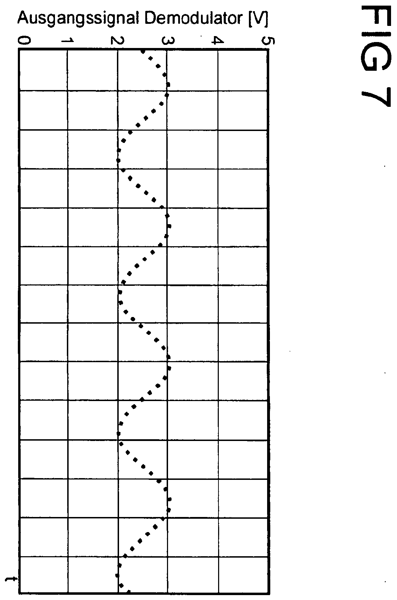

Wie bereits erwähnt, soll das Sensorsystem auch einen dichten Schaum erkennen. Dichter Schaum kann bspw. bei Blut als Messflüssigkeit entstehen, wenn Undichtigkeiten im mit dem Blasenfänger druckdicht verbundenen Schlauchsystem auftreten, in deren Folge Luft in den Flüssigkeits- oder Blutkreislauf eindringen kann. Aufgrund des Förderverhaltens des Gesamtsystems, wie einer Blutpumpe, ist der Anteil und Verteilung des Schaums über die Zeit nicht konstant. So ergibt sich über die Zeit eine Veränderung des gemessenen Feldes, die die gemessene komplexe Impedanz Z_{ges} verändert. In Figur 7 ist der Verlauf der Änderung der Phase abgebildet. Dieser Verlauf wird vom Demodulator aus der gemessenen komplexen Impedanz Z_{ges} gebildet und der Steuer- und Auswerteeinheit übergeben. Wenn die Auswerteeinheit derart schwankende Ergebnisse erhält, so kann eine entsprechende Warnung (optisch, akustisch, haptisch) vor Undichtigkeit an den Anwender ausgegeben werden, oder ein Behandlungsvorgang gestoppt oder nicht begonnen werden. Der Demodulator kann ebenfalls den Betrag der gemessenen komplexen Impedanz Z_{ges} bilden und der Steuer- und Auswerteeinheit übergeben.

Das Sensorsystem ist so eingerichtet, dass die einzelnen Messungen ausreichend häufig durchgeführt werden. Dabei liegt die Messhäufigkeit im Millisekunden- oder 1/10- Sekundenbereich.

Fig. 8 zeigt eine Möglichkeit der Halterung des Behälters 20 in der Aufnahme 30 des Sensorsystems. Dabei sind zwei Arme 24 in Drehpunkten 26 gegen die Aufnahme gelagert. Eine nicht dargestellte Federunterstützung sorgt dafür, dass der Behälter an die bereits beschriebenen Kontaktflächen gedrückt wird.

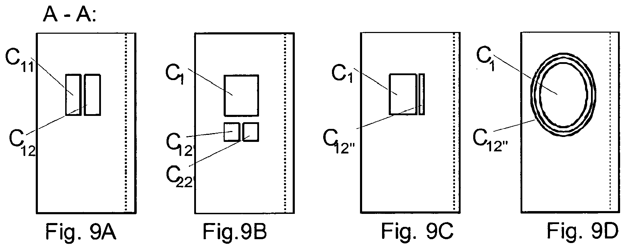

Fig. 9 zeigt unterschiedliche Varianten der Elektrodengestaltung, welches der Ansicht A - A der Fig. 4 entspricht. Gemäß Fig. 9A sind die Elektroden C₁₁ und C₁₂ gleich groß, welches der beschriebenen Hauptausführung entspricht, bei der diese gleichartigen Elektroden für die Pegelerkennung paarweise verwendet werden.

Alternativ können für die Pegelerkennung und die Koppelungserkennung unterschiedliche Elektroden verwendet werden. So ist gemäß Fig. 9B die Elektrode C₁ ausschließlich für die Pegelerkennung zuständig, während die Elektroden C_{11'} und C_{12'} für die Koppelung zuständig sind. Durch die unterschiedliche Ausrichtung in axialer Richtung des Behälters können die Messungen ggf. gleichzeitig durchgeführt werden, was natürlich eine andere, hier nicht näher erläuterte Analyse- und Auswerteeinheit erforderlich macht. Auch kann, wie in Fig. 9C und 9D dargestellt, eine Hauptelektrode C₁ zur Pegelerkennung verwendet werden, welche in den Messvorgängen der Koppelungsmessung im Verbund mit der entsprechenden Hilfselektrode C_{12"} arbeitet. Während bei Fig. 9C die Hilfselektrode C_{12"} sich zu einer Seite der Hauptelektrode C₁ erstreckt, ist bei Fig. 9D die Hauptelektrode oval (alternativ: rund) und die Hilfselektrode C_{12"} liegt koaxial nach außen umlaufend versetzt.

Vorzugsweise kann die Erfindung in medizinischen Geräten, wie einem Dialysegerät und insbesondere dessen Blasenfänger eingesetzt werden. Ein Blasenfänger dient hier primär zur Abscheidung von Luft aufgrund der Querschnittsaufweitung. Da der Abfluss aus dem Blasenfänger an dessen unterer Stirnseite liegt, werden mögliche Blasen von der weiter fließenden Flüssigkeit getrennt.

## Patentansprüche

1. Sensorsystem (10) zur kapazitiven Messung des Füllstands eines flüssigen Mediums (22) in einem Behälter, umfassend eine Aufnahme (30) an welcher zwei Kontaktbereiche zum Kontakt mit einer Außenfläche des aufgenommenen Behälters vorgesehen sind und mit zumindest je einer in den Kontaktbereichen angeordneten Pegelerkennungselektrode (C₁, C₂, C₁₁, C₁₂, C₂₁, C₂₂) zur kapazitiven Erkennung des Flüssigkeitspegels im Behälter, **dadurch gekennzeichnet, dass**
das Sensorsystem eine kapazitive Koppelungsmessvorrichtung (C₁₁, C₁₂, C₂₁, C₂₂) zur kapazitiven Bestimmung des ordnungsgemäßen Ankoppelns des Behälters in der Aufnahme aufweist, wobei Mittel zur Erzeugung von elektrischen Feldern für die Pegel- und Koppelungsmessung und Mittel zur Erfassung der komplexen Impedanz unterschiedlicher Pegelerkennungselektroden (C₁₁, C₁₂, C₂₁, C₂₂) nach Betrag, Phase und deren zeitlicher Änderung vorhanden sind.

2. Sensorsystem gemäß Anspruch 1, **dadurch gekennzeichnet, dass** die Pegelerkennungselektroden geteilte Elektrodenbereiche aufweisen und dass die Koppelungsmessvorrichtung die Koppelung über eine kapazitive Messung über die Elektrodenbereiche der jeweiligen Pegelerkennungselektrode erkennt.

3. Sensorsystem gemäß Anspruch 1, **dadurch gekennzeichnet, dass** die Kopplungsmessvorrichtung an jedem Kontaktbereich zwei Koppelelektroden (C11', C12', C21, C22) aufweist, die sich von den Pegelerkennungselektroden (C1, C2) unterscheiden.

4. Sensorsystem gemäß einem der vorangegangen Ansprüche, **dadurch gekennzeichnet, dass** eine Steuer- und Auswerteeinheit vorgesehen und dazu geeignet ist, im Fall, dass die Messung des Flüssigkeitspegels im Behälter einen ausreichenden Pegel erkennt und gleichzeitig die Kopplungsmessung eine unzureichende Kopplung erkennt, einen Voralarm auszulösen, um den Anwender auf eine unzureichende Kopplung hinzuweisen.

5. Sensorsystem gemäß einem der vorangegangen Ansprüche, **dadurch gekennzeichnet, dass** es eingerichtet ist, über die Pegelerkennungselektroden eine komplexe Gesamtimpedanz Z_{ges} zu bestimmen und über deren Auswertung die Pegelerkennung durchzuführen.

6. Sensorsystem gemäß Anspruch 5, **dadurch gekennzeichnet, dass** es eingerichtet ist, über Auswertung des Realteils der Gesamtimpedanz die Pegelerkennung durchzuführen.

7. Sensorsystem gemäß einem der vorangegangen Ansprüche, **dadurch gekennzeichnet, dass** es eingerichtet ist, über die Pegelerkennungselektroden eine komplexe Gesamtimpedanz Z_{ges} zu bestimmen und über einen Demodulator zeitliche Variationen der komplexe Gesamtimpedanz Z_{ges} zu erhalten und in einer Auswerteeinheit zusammen mit der komplexe Gesamtimpedanz Z_{ges} zur Bestimmung der Pegelerkennung eingesetzt zu werden, um so Inhomogenitäten wie z.B. einen Blasenanteil zu erkennen.

8. Sensorsystem gemäß einem der vorangegangenen Ansprüche, **dadurch gekennzeichnet, dass** der Behälter ein Blasenfänger eines Dialysegeräts, einer Infusions- oder Transfusionsapparatur ist.

9. Verfahren zum kapazitiven Messen des Füllstands eines flüssigen Mediums (22) in einem Behälter, bei dem der Behälter in einer Aufnahme des Sensorsystems aufgenommen wird und eine Pegelerkennungsvorrichtung (C₁, C₂, C₁₁, C₁₂, C₂₁, C₂₂) des Sensorsystems den Flüssigkeitspegel im Behälter kapazitiv ermittelt, **dadurch gekennzeichnet, dass** mit einer kapazitiven Koppelungsmessvorrichtung (C₁₁, C₁₂, C₂₁, C₂₂) das ordnungsgemäße Ankoppeln des Behälters in der Aufnahme bestimmt wird, wobei Mittel zur Erzeugung von elektrischen Feldern für die Pegel- und Koppelungsmessung und Mittel zur Erfassung der komplexen Impedanz unterschiedlicher Pegelerkennungselektroden (C₁₁, C₁₂, C₂₁, C₂₂) nach Betrag, Phase und deren zeitlicher Änderung vorhanden sind.

10. Verfahren gemäß Anspruch 9, **dadurch gekennzeichnet, dass** die Pegelerkennungsmessung und die Koppelungsmessung zeitlich versetzt durchgeführt werden.

11. Verfahren gemäß Anspruch 9 oder 10, **dadurch gekennzeichnet, dass** eine Steuer- und Auswerteeinheit vorgesehen ist, die im Fall, dass die Messung des Flüssigkeitspegels im Behälter einen ausreichenden Pegel erkennt und gleichzeitig die Kopplungsmessung eine unzureichende Kopplung erkennt, einen Voralarm auslöst, um den Anwender auf eine unzureichende Kopplung hinzuweisen.

12. Verfahren gemäß einem der Ansprüche 9 bis 11, **dadurch gekennzeichnet, dass** über die Pegelerkennungselektroden eine komplexe Gesamtimpedanz Z_{ges} bestimmt wird und über einen Demodulator zeitliche Variationen der komplexe Gesamtimpedanz Z_{ges} erhalten werden und in einer Auswerteeinheit zusammen mit der komplexe Gesamtimpedanz Z_{ges} zur Bestimmung der Pegelerkennung eingesetzt werden, um so Inhomogenitäten wie z.B. einen Blasenanteil zu erkennen.

13. Medizinisches Gerät, insbesondere ein Dialysegerät, mit einem Sensorsystem nach einem der Ansprüche 1-8.

## Claims

1. Sensor system (10) for capacitively measuring the fill level of a liquid medium (22) in a container, comprising a receptacle (30), which is provided with two contact regions for the contact with an exterior surface of the received container, and with at least one level detection electrode (C₁, C₂, C₁₁, C₁₂, C₂₁, C₂₂) disposed in each of the contact regions for capacitively detecting the liquid level in the container, **characterised in that** the sensor system has a capacitive coupling measuring device (C₁₁, C₁₂, C₂₁, C₂₂) for capacitively determining correct coupling of the container in the receptacle, wherein means are present for generating electric fields for the level and coupling measurement and means are present for capturing the complex impedance of different level detection electrodes (C₁₁, C₁₂, C₂₁, C₂₂) according to magnitude, phase and their change over time.

2. Sensor system according to claim 1, **characterised in that** the level detection electrodes have divided electrode regions and that the coupling measuring device detects the coupling by way of a capacitive measurement over the electrode regions of the respective level detection electrode.

3. Sensor system according to claim 1, **characterised in that** the coupling measuring device has in each contact region two coupling electrodes (C11', C12', C21, C22), which differ from the level detection electrodes (C1, C2).

4. Sensor system according to any one of the preceding claims, **characterised in that** a control and evaluation unit is provided and is suitable to trigger a pre-alarm, when the measurement of the liquid level in the container detects a sufficient level and at the same time the coupling measurement detects an insufficient coupling, in order to draw the user's attention to an insufficient coupling.

5. Sensor system according to any one of the preceding claims, **characterised in that** it is configured to determine a complex total impedance Zₜₒₜ via the level detection electrodes and to perform the level detection via the evaluation of said total impedance.

6. Sensor system according to claim 5, **characterised in that** it is configured to perform the level detection via the evaluation of the real part of the total impedance.

7. Sensor system according to any one of the preceding claims, **characterised in that** it is configured to determine a complex total impedance Zₜₒₜ via the level detection electrodes and to obtain variations over time in the complex total impedance Zₜₒₜ via a demodulator, and to be used in an evaluation unit together with the complex total impedance Zₜₒₜ for determination of the level detection, in order by this means to recognise inhomogeneities such as for example a bubble fraction.

8. Sensor system according to any one of the preceding claims, **characterised in that** the container is a bubble catcher of a dialysis device, an infusion apparatus or a transfusion apparatus.

9. Method for capacitively measuring the fill level of a liquid medium (22) in a container, wherein the container is received in a receptacle of the sensor system and a level detection device (C₁, C₂, C₁₁, C₁₂, C₂₁, C₂₂) of the sensor system determines capacitively the liquid level in the container, **characterised in that** the correct coupling of the container in the receptacle is determined using a capacitive coupling measuring device (C₁₁, C₁₂, C₂₁, C₂₂), wherein means are present for generating electric fields for the level and coupling measurement and means are present for capturing the complex impedance of different level detection electrodes (C₁₁, C₁₂, C₂₁, C₂₂) according to magnitude, phase and their change over time.

10. Method according to claim 9, **characterised in that** the level detection measurement and the coupling measurement are chronologically staggered.

11. Method according to claim 9 or 10, **characterised in that** a control and evaluation unit is provided, which triggers a pre-alarm, when the measurement of the liquid level in the container detects a sufficient level and at the same time the coupling measurement detects an insufficient coupling, in order to draw the user's attention to an insufficient coupling.

12. Method according to any of claims 9 to 11, **characterised in that** a complex total impedance Zₜₒₜ is determined via the level detection electrodes and variations over time in the complex total impedance Zₜₒₜ are obtained via a demodulator and to be used in an evaluation unit together with the complex total impedance Zₜₒₜ for determination of the level detection, in order by this means to recognize inhomogeneities such as for example a bubble fraction.

13. Medical device, in particular a dialysis device, with a sensor system according to any of claims 1-8.

## Revendications

1. Système capteur (10) pour la mesure capacitive du niveau de remplissage d'un milieu liquide (22) dans un récipient, comprenant un logement (30) au niveau duquel deux zones de contact sont prévues pour le contact avec une surface extérieure du récipient logé et avec au moins respectivement une électrode de détection de niveau (C₁, C₂, C₁₁, C₁₂, C₂₁, C₂₂), agencée dans les zones de contact pour la détection capacitive du niveau de liquide dans le récipient, **caractérisé en ce que**
le système capteur présente un dispositif de mesure de couplage capacitif (C₁₁, C₁₂, C₂₁, C₂₂) pour la détermination capacitive de l'accouplement correct du récipient dans le logement, dans lequel des moyens de génération de champs électriques pour la mesure de niveau et de couplage et des moyens de capture de l'impédance complexe de différentes électrodes de détection de niveau (C₁₁, C₁₂, C₂₁, C₂₂) en fonction de la valeur, de la phase et de leur modification temporelle sont présents.

2. Système capteur selon la revendication 1, **caractérisé en ce que** les électrodes de détection de niveau présentent des zones d'électrodes divisées et que le dispositif de mesure de couplage détecte le couplage par le biais d'une mesure capacitive par le biais des zones d'électrodes de l'électrode de détection de niveau respective.

3. Système capteur selon la revendication 1, **caractérisé en ce que** le dispositif de mesure de couplage présente au niveau de chaque zone de contact deux électrodes de couplage (C11', C12', C21, C22), qui se distinguent des électrodes de détection de niveau (C1, C2).

4. Système capteur selon l'une quelconque des revendications précédentes, **caractérisé en ce qu'**une unité de commande et d'évaluation est prévue et adaptée, dans le cas où la mesure du niveau de liquide dans le récipient détecte un niveau suffisant et en même temps la mesure de couplage détecte un couplage insuffisant, pour déclencher une préalarme pour indiquer un couplage insuffisant à l'utilisateur.

5. Système capteur selon l'une quelconque des revendications précédentes, **caractérisé en ce qu'**il est aménagé pour déterminer une impédance totale complexe Zₜₒₜ par le biais des électrodes de détection de niveau et effectuer la détection de niveau par le biais de son évaluation.

6. Système capteur selon la revendication 5, **caractérisé en ce qu'**il est aménagé pour effectuer la détection de niveau par le biais de l'évaluation de la part réelle de l'impédance totale.

7. Système capteur selon l'une quelconque des revendications précédentes, **caractérisé en ce qu'**il est aménagé pour déterminer une impédance totale complexe Zₜₒₜ par le biais des électrodes de détection de niveau et pour obtenir des variations temporelles de l'impédance totale complexe Zₜₒₜ par le biais d'un démodulateur et pour être utilisé dans une unité d'évaluation conjointement avec l'impédance totale complexe Zₜₒₜ pour la détermination de la détection de niveau afin de détecter ainsi des défauts d'homogénéité, par exemple une part de bulles.

8. Système capteur selon l'une quelconque des revendications précédentes, **caractérisé en ce que** le récipient est un attrape-bulles d'un dialyseur, d'un appareillage de perfusion ou de transfusion.

9. Procédé pour la mesure capacitive du niveau de remplissage d'un milieu liquide (22) dans un récipient, dans lequel le récipient est logé dans un logement du système capteur et un dispositif de détection de niveau (C₁, C₂, C₁₁, C₁₂, C₂₁, C₂₂) du système capteur détermine de manière capacitive le niveau de liquide dans le récipient, **caractérisé en ce que** l'accouplement correct du récipient dans le logement est déterminé avec un dispositif de mesure de couplage capacitif (C₁₁, C₁₂, C₂₁, C₂₂), dans lequel des moyens de génération de champs électriques pour la mesure de niveau et de couplage et des moyens de capture de l'impédance complexe de différentes électrodes de détection de niveau (C₁₁, C₁₂, C₂₁, C₂₂) en fonction de la valeur, de la phase et de leur modification temporelle sont présents.

10. Procédé selon la revendication 9, **caractérisé en ce que** la mesure de détection de niveau et la mesure de couplage sont réalisées décalées dans le temps.

11. Procédé selon la revendication 9 ou 10, **caractérisé en ce qu'**une unité de commande et d'évaluation est prévue, qui, dans le cas où la mesure du niveau de liquide dans le récipient détecte un niveau suffisant et en même temps la mesure de couplage détecte un couplage insuffisant, déclenche une préalarme pour indiquer un couplage insuffisant à l'utilisateur.

12. Procédé selon l'une quelconque des revendications 9 à 11, **caractérisé en ce qu'**une impédance totale complexe Zₜₒₜ est déterminé par le biais des électrodes de détection de niveau et des variations temporelles de l'impédance totale complexe Zₜₒₜ sont obtenues par le biais d'un démodulateur et sont utilisées dans une unité d'évaluation conjointement avec l'impédance totale complexe Zₜₒₜ pour la détermination de la détection de niveau afin de détecter ainsi des défauts d'homogénéité, par exemple une part de bulles.

13. Appareil médical, en particulier un dialyseur, avec un système capteur selon l'une quelconque des revendications 1-8.
